# EUROPEAN PATENT SPECIFICATION

(11) **EP 3 369 451 B1**
(45) Date of publication and mention of the grant of the patent: **29.04.2020**
(21) Application number: 17305225.9
(22) Date of filing: 02.03.2017
(51) Int. Cl.: A61M 16/06

(54) **FRAMELESS RESPIRATORY MASK**
RAHMENLOSE ATEMMASKE
MASQUE RESPIRATOIRE SANS CADRE

(43) Date of publication of application: 05.09.2018
(73) Proprietor: Air Liquide Medical Systems, 92160 Antony (FR)
(72) Inventor: ALBERICI, Luca, 25030 RONCADELLE (BS) (IT); MASSERDOTTI, Fulvio, 25075 BRESCIA (IT); SANDONI, Giuseppe, 25124 BRESCIA (IT); BUGATTI, Ottorino, 25068 Sarezzo (Bs) (IT)
(74) Representative: Pittis, Olivier

(56) References cited:
- EP-A1- 2 954 921
- US-A1- 2014 090 649
- US-A1- 2016 279 371
- US-A1- 2016 325 067

## Description

The invention concerns a frameless respiratory mask, in particular a frameless nasal mask or a frameless facial mask, comprising a front shroud, a ring and a flexible cushion coupled together, but not comprising any mask body or frame. The frameless respiratory mask is intended in particular for adult or pediatric uses in the treatment of respiratory conditions or diseases affecting adults, children, toddlers, infants or newborns.

Respiratory masks are commonly used for delivering a flow of breathable gas, such as air under pressure (>1 bar), for or to assist in patient respiration. A mask assembly typically comprises a rigid or semi-rigid hollow shell or body, usually made of polymer, defining a breathing chamber that receives at least a part of the patient's nose and further comprising a soft face-contacting cushion that comes into contact with the patient's face and conforms to the various facial contours of the patient face. The cushion is coupled to the hollow shell or body. The cushion is usually made of soft, resilient elastomeric material, such as soft silicone or similar material.

The hollow shell typically receives a gas supply line which delivers a respiratory gas, such as air under pressure, into the breathing chamber of the shell. An example of a mask of this kind is given by EP-A-462701.

A mask additionally may have a forehead support and a headgear for correctly positioning, maintaining and/or securing the mask on the head of a patient by means of straps or similar. The headgear that can be adjusted to pull the mask against the face with sufficient force to achieve a gas tight seal between the mask and the wearer's face as taught by EP-A-462701, EP-A-874667, EP-A-1972357 or WO-A-00/57942.

The forehead support and headgear connections can be arranged on a front shroud that is connected to the mask shell or body, as disclosed by EP-A-2708258 or US-A-2007/0044804. Thus, EP-A-2708258 teaches a mask system comprising a frame or mask body defining a breathing chamber, a cushion provided to the frame and adapted to form a seal with the patient's face, and a shroud provided to the frame or mask body and adapted to attach a headgear. The frame comprises a collar surrounding an opening for receiving an elbow connector. A retaining mechanism establishes a positive connection between the shroud and the frame or mask body, said mechanism comprising one or more snap-fingers structured to engage the collar with a snap-fit.US-A-2007/0044804 also discloses a similar architecture.

Further, US-A-2016/0279371 discloses a respiratory mask comprising a mask frame, also called exoskeleton, a sealing arrangement, such as a flexible cushion, a ring arranged in-between, and an elbow connector. The role of the ring is to provide an interface between the cushion and the elbow connector, i.e. to allow the elbow connector to connect to the cushion. The ring can comprise venting holes.

The problem with such masks is that their architecture is too complex for many patients. This leads to some difficulties for them to correctly assemble the different components of the mask, in particular for well-connecting the shroud, the mask body and the cushion together resulting in patient discomfort, and afterwards to gas leaks due to wrong assembling of the components,. This may arise, when a patient has to disassemble and then re-assemble some components of the mask, for instance in the case where a patient needs to clean up the inner parts of the mask.

The goal of the present invention is to provide an improved mask, especially a frameless nasal or facial mask, having a straightforward architecture for the patients, thereby avoiding or minimizing the risks of bad assembling of different components and of related leak and discomfort issues.

In other words, the goal of the present invention is to provide an improved respiratory mask architecture, especially a nasal or facial mask, that is simple to assemble, light and comfortable to wear, thanks to a simplified architecture, thereby ensuring efficient positioning and securing of the mask on the patient's face, good comfort of use for the patients and a efficient gas tightness, i.e. seal, preventing the escape of gas that may result from a poor coupling of the components of the mask.

The solution of the present invention concerns a frameless respiratory mask, in particular a frameless nasal mask, comprising:
- a shroud comprising an upper arm projecting upwardly and two lateral arms projecting laterally, the upper arm and the two lateral arms being arranged around a central opening traversing the shroud, the upper arm and the two lateral arms each comprising a free end with headgear connection structures adapted for connecting a headgear thereto,
- a rigid ring traversed by an inner passage, and
- a flexible cushion defining a breathing chamber, said breathing chamber comprising a gas orifice, and a rear opening adapted for receiving at least a part of a patient's face,
and wherein:
- the rigid ring is sandwiched between the shroud and the flexible cushion, and
- the gas orifice of the flexible cushion is coaxially arranged and in fluid communication with the central opening of the shroud,
characterized in that:
i) the rigid ring is integrally and non-removably attached to the flexible cushion, and
ii) the shroud is further removably attached directly to the rigid ring.

In other words, according to the present invention and in contrast with the prior art, the shroud is directly removably coupled or fixed to the ring and/or to the flexible cushion, preferably attached to the ring, in lieu of being attached to a rigid mask body or frame. In the frameless mask of the invention, there is no mask body or frame.

Such a configuration further provides an easy connecting by a user of the cushion to the shroud, i.e. limited risks of misalignment or misconnecting.

The frameless mask of the present invention is advantageous as it provides a respiratory mask that is, on the one hand, easier to assemble and/or to disassemble and, on the other hand, lighter (as less components are need) and hence more comfortable for the patients, while at the same time its sealing properties are not negatively affected.

### Shroud

The shroud of the frameless mask according to the present invention can further comprise one or more of the following additional features:
- the shroud is coupled/attached directly to the ring and/or to the flexible cushion by means of connecting structures cooperating together, said connecting structures being carried by the shroud and the ring and/or the flexible cushion.
- advantageously, the shroud is removably coupled/attached directly to the ring, i.e. the shroud can be attached to the ring in a removable manner so as to be detachable therefrom for cleaning or maintenance operations, replacement, or the like, of the shroud, the ring and/or the cushion.
- the connecting structures are carried by the shroud and the rigid ring so as to hold the shroud attached directly to the rigid ring.
- the shroud comprises an outer wall comprising an annular free end, said annular free end comprising at least one abutment (i.e. one or several abutments) projecting outwardly, i.e. the at least one abutment projects away from the peripheral outer surface of the annular outer wall of the shroud.
- the shroud comprises an inner wall delimiting the central opening and the outer wall comprising an annular free end carrying the at least one abutment, said inner wall and outer wall being spaced apart from each other, said inner wall and outer wall sandwiching a collar surrounding the gas orifice of the flexible cushion, thereby ensuring a gas tightness between the inner and outer walls and the collar so that no gas is able to escape in-between, i.e. sealing.
- preferably, said inner wall and outer wall of the shroud are coaxially arranged.
- the inner wall and the outer wall of the shroud are spaced by an annular chamber, i.e. avoid spacing, the collar of the cushion being inserted into said annular chamber of the shroud, and squeezed between said inner wall and outer wall of the shroud, thereby ensuring a gas tightness in-between so that no gas is able to escape.
- the shroud comprising the upper arm and the two lateral arms is molded in one piece and made of plastic material.
- the holding arm and the two lateral arms of the shroud are made of a flexible plastic material so that are able to bent, in use, toward the patient's face, in particular upon a traction force resulting from an adjustment of the tightness of the headgear.
- the shroud, including the holding arm and the two lateral arms, is made of a plastic material, i.e. polymer, chosen among polycarbonate (PC), polypropylene (PP), nylon, or the like.
- the holding arm and of the two lateral arms have an elongated shape.
- the holding arm and of the two lateral arms have a band or ribbon shape. Other shapes are of course also possible.
- the holding arm has a length of about 2 to 12 cm, preferably of about 2 to 8 cm.
- each lateral arm has a length of about 3 to 10 cm, preferably of about 3 to 6 cm.
- the shroud comprises a central opening having a diameter of between 1 and 4 cm.

### Cushion

The cushion of the frameless mask according to the present invention can further comprise one or more of the following additional features:
- the flexible cushion comprises a collar surrounding the gas orifice.
- the collar of the cushion comprises an annular bump or boss, projecting inwardly, said annular boss being squeezed between said inner wall and outer wall of the shroud, when the shroud is fixed to the ring.
- the frameless mask is a nasal mask comprising a cushion sized for covering at least part of the nose region of a user, when the mask is worn by said user.
- alternatively, the frameless mask is a facial mask comprising a cushion sized for covering at least part of the nose region and of the mouth region of a user, when the mask is worn by said user.
- the breathing chamber of the cushion comprises a rear opening adapted for receiving at least a part of a patient's nose, said rear opening being bordered at its periphery by a flexible membrane.
- the rear opening of the cushion is bordered at its periphery by a flexible membrane forming a flexible sealing skirt.
- the cushion further comprises a front opening or front orifice in fluid communication with the breathing chamber.
- the cushion further comprises a collar delimiting the front orifice.
- the collar surrounds the front orifice of the flexible cushion.
- the collar projects outwardly.
- the flexible cushion is made of silicone or the like.
- the flexible cushion comprises an inner chamber, i.e. a respiratory chamber, in fluid communication with the gas passage of a hollow gas connector, in particular a curved or elbow connector.
- the flexible cushion comprises a rear aperture in fluid communication with the inner chamber and adapted for receiving at least part of the patient's nose, when the patient wears the mask. The rear aperture of the flexible cushion is also called "nose aperture" or "nose opening".
- the aperture comprises a peripheral border configured to ensure a gas tightness between the mask and the patient's face, in particular the nasal region.
- the flexible cushion comprises a peripheral border comprising one or several membranes arranged around along or part of the peripheral border, i.e. at the periphery of the aperture of the respiratory inner chamber.
- at least one membrane forms a flexible skirt around the along or part of the periphery of the aperture of the respiratory chamber so as to ensure an efficient gas tightness while being in contact with the patient's face, when the latter wears the mask, preferably all along the periphery of the aperture of the respiratory chamber.
- the membrane(s) is (are) be molded in one piece with the rest of the cushion.
- the cushion is made of a resilient soft or semi-soft material, i.e. of silicone or similar.
- the cushion is configured and sized for coming into contact, when the mask is worn by the user or patient, with regions of the patient's nose so as to ensure an efficient gas sealing with the patient's face. Those regions comprise the nasal bridge region or the area at the junction of the bone and cartilage, and the lateral nose are those located on each side of the nose, and either the region between nose and the upper lip of the mouth, or the region between lower lip and chin.

### Ring

The ring of the frameless mask according to the present invention can further comprise one or more of the following additional features:
- the ring is non-removably attached to the flexible cushion, i.e. the ring cannot be easily detached and is not supposed to be detachable from the flexible cushion by a user. In other others, the ring forms with the shroud an integrated structure.
- the ring is non-removably snap-fitted or similar to the flexible cushion.
- the rigid ring has a generally annular or tubular shape.
- the rigid ring is made of plastic material.
- the rigid ring is made of plastic material chosen among polyamide (PA), polycarbonate (PC) and polypropylene (PP), or the like.
- the gas orifice of the flexible cushion, the central opening of the shroud and the inner passage of the rigid ring are coaxial.
- the rigid ring is traversed by a central orifice.
- the ring comprises a central orifice having a diameter of between 1 and 4 cm.
- the rigid ring comprises an inner annular wall and at least one groove, i.e. at least one lodging, arranged in said an inner annular wall, said and at least one groove comprising a front border.
- preferably, said at least one groove is or comprises an annular groove.

### Connecting structure

The connecting structures of the frameless mask according to the present invention can further comprise one or more of the following additional features:
- the connecting structures for removably and firmly attaching the shroud to the ring and/or cushion, preferably to the rigid ring, comprise snap-fit connecting structures, threaded connecting structures, bayonet connecting structures, plug-in structures or the like.
- the shroud comprises an inner wall delimiting the central opening and an outer wall, spaced apart from each other, said inner wall and outer wall sandwiching, i.e. squeezing, the collar surrounding the front orifice of the flexible cushion so as to ensure a gas tightness between the shroud and the flexible cushion.
- the inner and outer walls are arranged face-to-face.
- the inner and outer walls form coaxially-arranged tubular structures.
- the inner and outer walls are separated by an annular chamber, i.e. the annular chamber is delimited by the coaxially-arranged tubular structures.
- the at least one abutment of the shroud is lodged into the at least one groove, preferably an annular groove, of the rigid ring and further abuts against said front border of the rigid ring, for thereby holding the shroud integral with the rigid ring, i.e. firmly attached to the ring, when the shroud is mounted on the mask.
- the at least one abutment of the shroud forms teeth or teeth-like structures.
- the at least one groove of the rigid ring forms one or several seats or seat-like structures.
- the shroud is firmly and removably coupled/attached directly to the ring by means of one or several teeth carried by the shroud cooperating with one or several seats carried by the rigid ring.
- the collar surrounding the front orifice of the flexible cushion is positioned into the annular chamber and integrally maintained therein by the inner and outer walls, i.e. by the coaxially-arranged tubular structures.
- the inner wall and the outer wall are spaced by an annular chamber, the collar of the cushion being inserted and hold into said annular chamber.

### Elbow connector

The elbow connector of the frameless mask according to the present invention can further comprise one or more of the following additional features:
- a hollow curved connector is fixed to the shroud, i.e. held in position on the shroud.
- the hollow curved connector is detachably fixed to the shroud.
- the hollow curved connector is rotatable with respect to said shroud.
- a tubular element is detachably fixed to the hollow curved connector, and rotatable with respect to said hollow curved connector.
- the tubular element is configured for connecting a gas line thereto. In other words, the tubular element is adapted for receiving a flexible gas conduct or hose that feeds a respiratory gas under pressure to the respiratory mask. The gas is fed into the flexible gas conduct or hose by a gas source, such as a medical ventilator.
- the hollow curved connector is in fluid communication with the breathing chamber of the cushion thereby allowing gas to circulate from the hollow curved connector to the cushion, or vice versa.
- the hollow curved connector has general "L" shape or a similar shape.
- the hollow curved connector comprises venting holes.
- the venting holes are arranged in a cover attached to an aperture of the hollow curved connector, preferably the aperture is positioned in the elbow portion of the hollow curved connector.
- the hollow curved connector comprises a proximal end that is inserted and held in the central opening of the shroud, when the hollow curved connector is attached to the shroud of the mask.
- the proximal end of the hollow curved connector has a cylindrical or tronconical shape and comprises two external lips, spaced from each other, projecting outwardly around the outer periphery of the proximal end of the connector so as to form annular abutments that sandwich the inner wall of the central opening of the shroud.

### Headgear

The headgear of the frameless mask according to the present invention can further comprise one or more of the following additional features:
- the headgear further comprises a headgear attached to the headgear connection structures of the holding arm and/or at least one of the two lateral arms.
- the headgear further comprises a headgear comprising straps. The straps are connected to the holding arm and of the two lateral arms. The headgear is used for maintaining and securing the mask in a desired position on the head of the patient, in particular the facial region of the patient.
- the straps of the headgear are made of polymer material or fabric material, or both.

The invention also concerns a respiratory assembly comprising a gas delivery device, such as a medical ventilator, and a frameless mask according to the present invention, preferably a nasal mask. The gas delivery device is connected to the frameless mask by means of a gas line, such as a flexible hose. Typically, the gas line is connected to the tubular connecting piece fixed to the hollow curved connector, said hollow curved connector being itself fixed to the frameless mask.

A non-limitative embodiment of a respiratory mask, especially a nasal mask, according to the present invention is shown in the enclosed Figures, among which:
- Figure 1 represents a front lateral view of a frameless mask according to one embodiment of the present invention,
- Figure 2 is an exploded view of the mask of Figure 1,
- Figure 3 is an exploded view of the cushion and ring of the mask of Figure 1,
- Figures 4 is a front view of the mask of Figure 1,
- Figure 5 is a lateral cross-sectional representation of the mask of Figure 4,
- Figure 6 is an enlarged view of the "C" area of Figure 5,
- Figure 7 is cross-sectional view along line A-A of the mask of Figure 4; and
- Figure 8 is an enlarged view of the "D" area of Figure 7.

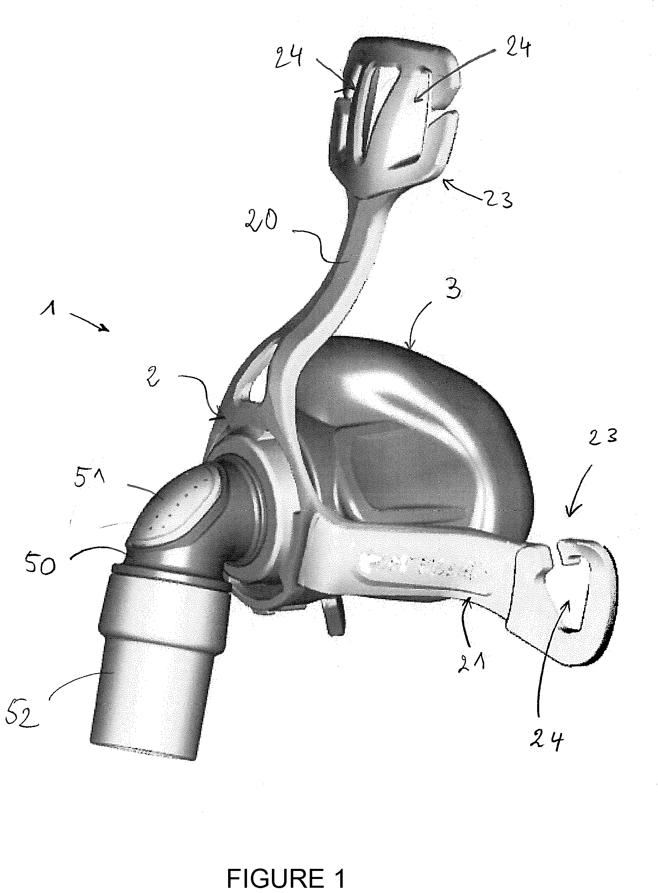

The present invention proposes a new structure of a respiratory mask as shown in Figures 1-8 that illustrate one embodiment of a frameless mask according to the present invention, in particular a frameless nasal mask.

Generally speaking, a frameless respiratory mask 1 according to the present invention comprises two main parts assembled together, namely a shroud 2 and a flexible cushion 3, and a rigid ring 4 sandwiched between said shroud 2 and flexible cushion 3.

As explained below, the ring 4 is firmly held in place by the cushion 3, whereas the shroud 2 is removably attached directly to the ring 4. No additional frame or mask body is needed for connecting the different components.

Indeed, the mask 1 of the present invention does not comprise any rigid mask body or "frame" arranged between the shroud 2 and the flexible cushion 3, as opposed to the masks disclosed by EP-A-2708258 and US-A-2007/0044804.

This leads to a frameless mask 1 having a simple and lighter structure, and further facilitates the assembling/disassembling of the different components of the mask 1, in particular of the shroud 2, by the users, namely the patients, especially during daily cleaning operations. Such a frameless mask 1 further exhibits a good comfort as it is lighter and has a low risk of gas leaks.

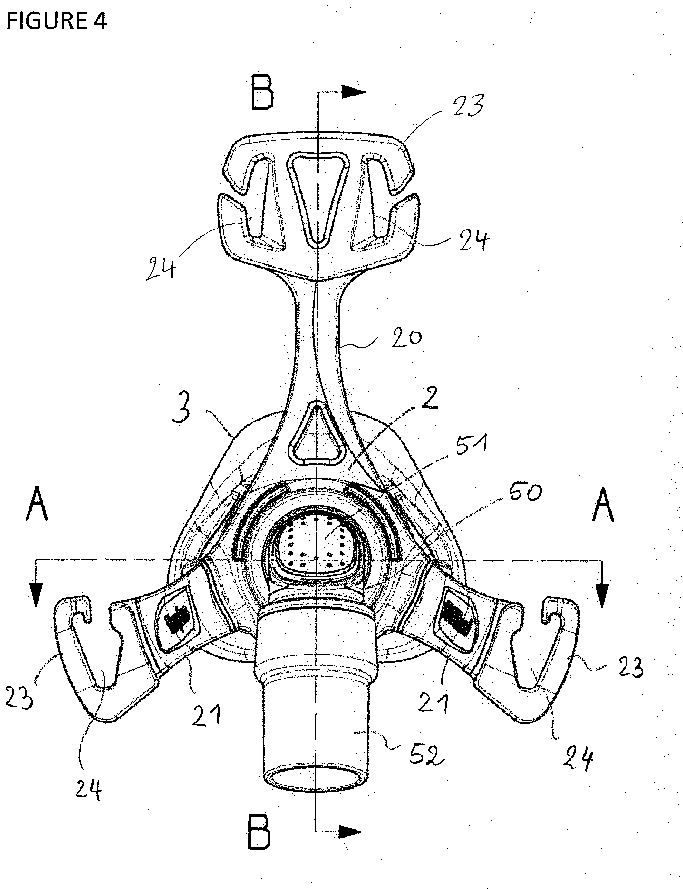

As shown in Figures 1, 2, 4 and 5, the frameless respiratory mask 1 of the present invention comprises a shroud 2 comprising an upper arm 20 projecting upwardly, i.e. about vertically, and two lateral arms 21 projecting laterally, in opposite directions, i.e. one toward the right side of the mask 1 and the other toward the left side of the mask 1 as shown in Figure 4. The upper arm 20 and the two lateral arms 21 are arranged around a central opening 22 traversing the shroud 2.

The holding arm 20 projecting upwardly (i.e. almost vertically) may also constitute a frontal support that comes into contact with the patient's forehead, whereas the two opposite lateral arms or wings 21 that project laterally may also constitute right and left cheek supports, when the mask is worn by a patient.

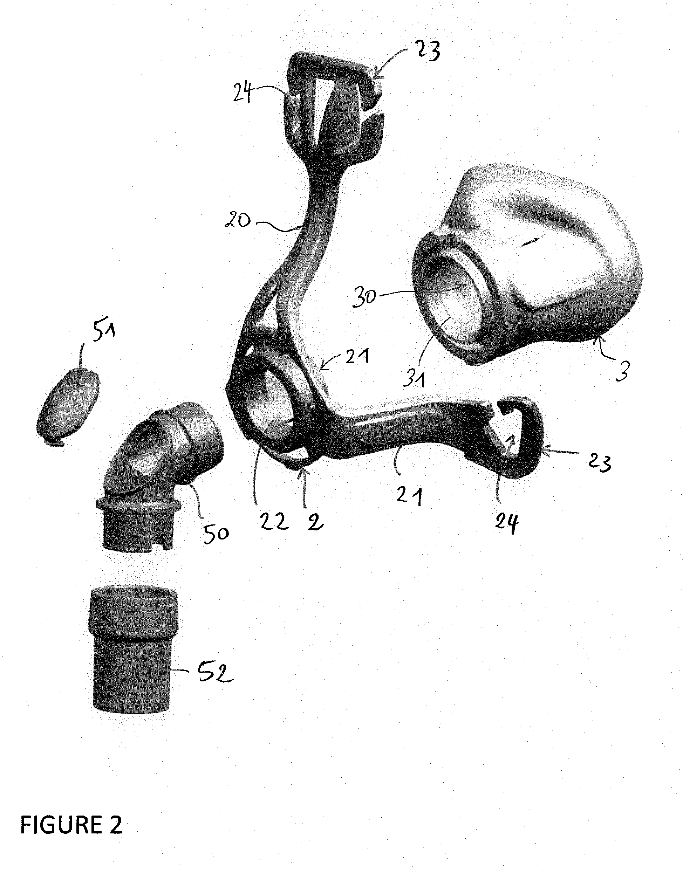

As shown in Figures 1 and 2, the two lateral arms 21 and the holding arm 20 of have elongated shapes, such as band or ribbon-like shapes, and are slightly incurved so as to better match some of the contours of the cushion 3, i.e. configured or shaped so as to better conform to the external profile of the flexible cushion 3.

Further, the upper arm 20 and the two lateral arms 21 each comprise a free end 23 with headgear connection structures 24, such as slots or hooks, adapted for connecting a headgear thereto (not shown).The headgear (not shown) is used for maintaining and securing the mask 1 in a desired position on the head of the patient. Generally, the straps of headgears are made of polymer or fabric materials.

Preferably, the shroud 2 is made of plastic material and molded in one piece. Nevertheless, it can be also made from several sub-units assembled together and made integral by means of any available fixing technique, for instance they can be glued, thermo-welded or similarly affixed to one another.

For example, the central opening 22 of the shroud 2 can have an inner diameter of about between 1 and 4 cm, whereas the two lateral arms 21 and the holding arm 20 can have lengths of about between 2 and 15 cm.

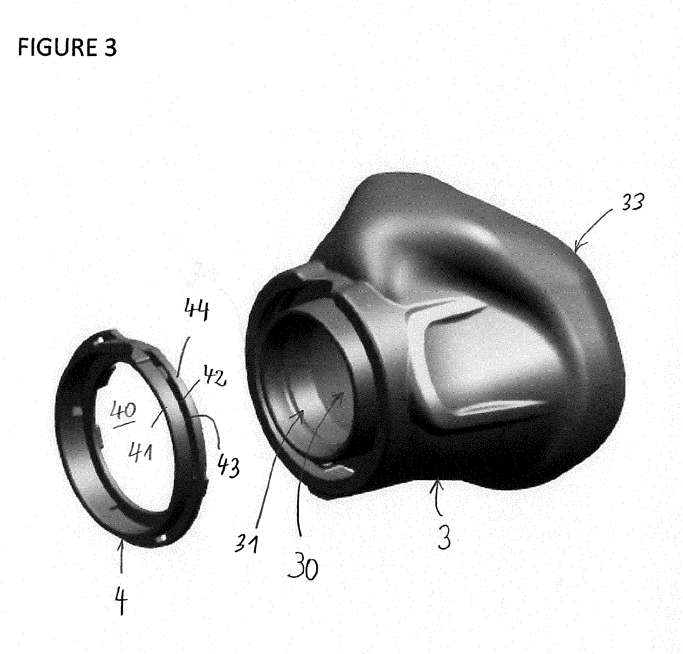

A rigid ring 4 traversed by an inner passage 40 is sandwiched between the shroud 2 and the flexible cushion 3, as illustrated in Fig. 3, 5 and 6.

Preferably, the rigid ring 4 is also made of plastic material. The main role of this ring 4 is to facilitate the quick coupling of the shroud 2 and the cushion 3 by a user, for example for daily cleaning operations or the like.

Typically, according to the present invention, the plastic material or polymer material that is used for manufacturing the shroud 2 and/or the ring 4 can be polypropylene (PP), polycarbonate (PC) or nylon, or similar materials.

The mask 1 further comprises a flexible cushion 3, preferably made of silicone, defining a breathing chamber 30 that comes into contact, in use, with the patient's face.

The shroud 2 is directly coupled to the ring 4, in a removable manner, i.e. so that it can be detached from the ring 4 for cleaning operations or the like. The rigid ring 4 is itself firmly held in place by the body of the flexible cushion 3 by a tight connection, such as snap-fitting, threading, a bayonet system or any other connection system, including over-molding. Preferably, the ring 4 cannot be detached from the cushion 3.

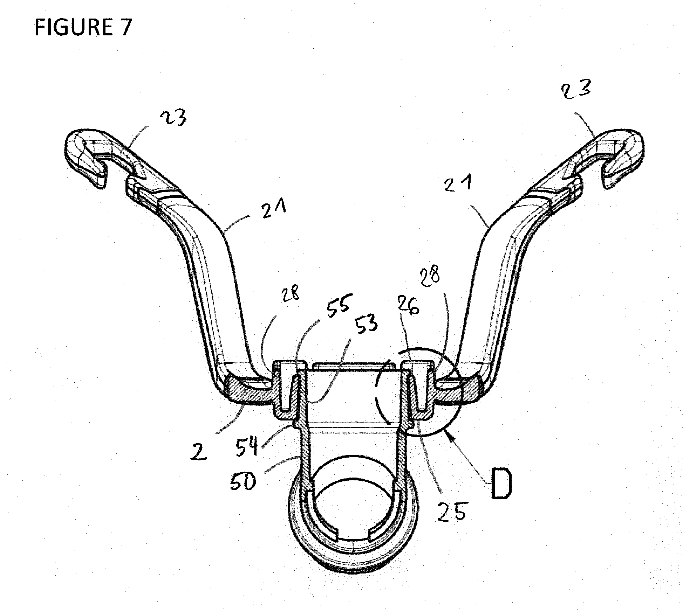

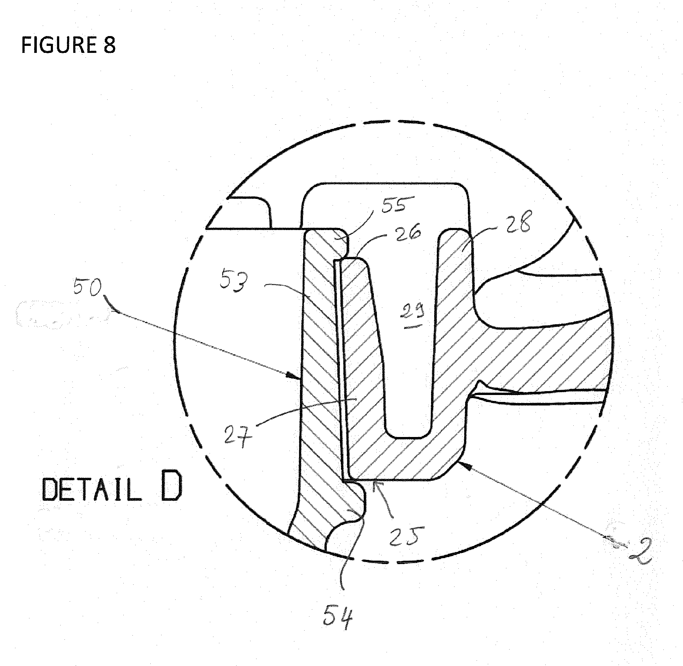

The breathing chamber 30 of the cushion 3 comprises a front orifice 31, i.e. a gas inlet orifice, and a rear opening 32 adapted for receiving at least a part of the patient's face. A respiratory gas, such as air, can enter into the breathing chamber 30 of the cushion 3 passing through the gas orifice 31, whereas gases expired by the patient, that are enriched in CO₂, can exit of the breathing chamber 30 by said gas orifice 31 and afterwards can be vented to the atmosphere by venting holes 51 arranged in an elbow tubular connector 50 connected to the mask 1 as shown in Figures 1 and 2, and detailed hereafter in connection with Fig. 7 and 8.

The venting holes 51 can be arranged directly through the wall of the elbow tubular connector 50 or can be arranged in an independent element, such as a cover or the like, as shown in Fig. 1 and 2, that is designed and a adapted for being attached to an aperture arranged in the wall of the elbow tubular connector 50.

The venting holes 51 have preferably a tronconical-shape passage for the gas with an inner diameter greater than an outer diameter.

Further, the front orifice 31 of the flexible cushion 3 is coaxially arranged and in fluid communication with the central opening 22 of the shroud 2 so that the gas can travel through said front orifice 31 and central opening 22.

The elbow tubular connector 50 is connected to the shroud 2 of the frameless mask 1 as detailed hereafter.

Typically, the flexible cushion 3 is a tridimensional hollow flexible structure forming a respiratory inner chamber or breathing chamber 30 with a rear opening 32, also called nose aperture, conformed and sized for receiving at least part of the patient's face, when the patient wears the mask, so that the patient can breathe the respiratory gas contained in said breathing chamber 30.

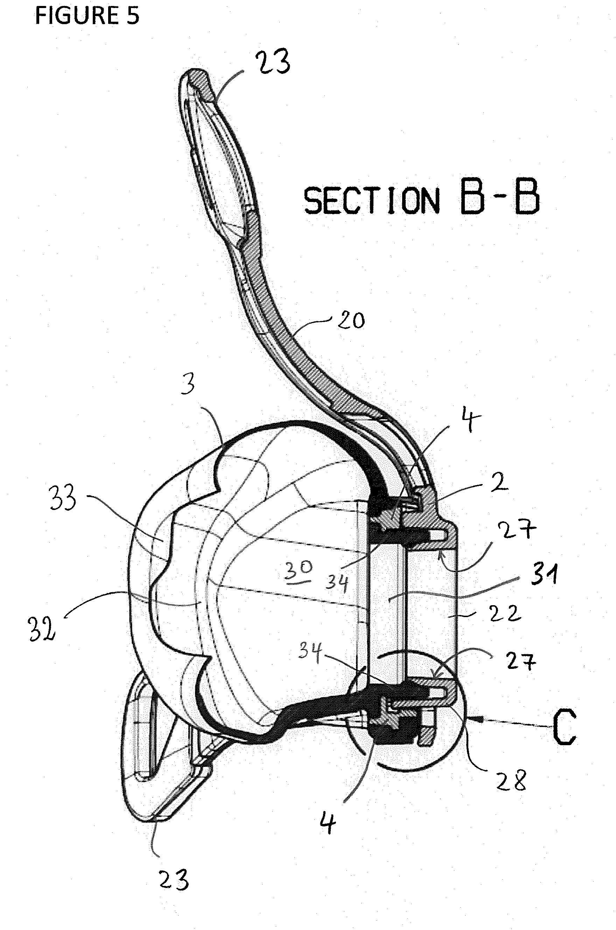

In order to provide efficient gas tightness (i.e. seal) and/or good comfort for the patient, the rear opening 32 of the cushion 3 is delimited by a flexible membrane 33, as shown in Fig. 5, that comes into contact with the patient's face and matches his/her facial morphology. The flexible membrane 33 is arranged preferably all along the border of the rear opening 32 thereby constituting a kind of soft flexible skirt delimiting the rear opening 32 of the cushion 3. In the embodiment shown in Fig. 5, a unique membrane 33 is arranged around the rear opening 32 of the cushion 3, but, in alternative embodiments, several membranes 2 may be superimposed.

Generally speaking, the cushion 3 can have any tridimensional form or shape (from a rear view) that matches the contours of the patient's face, especially in the nasal regions, such as trapezoidal, triangular or saddle shapes.

When the frameless nasal respiratory mask 1 of Figures 1 and 2 is worn by the patient, i.e. when the cushion 3 receives the patient's nose, when said patient introduces his/her nose into the internal volume of the breathing chamber 30 and breathes the gas contained therein, the membrane 33 of the cushion 3 that is arranged around the peripheral border of the rear opening 32, comes into contact roughly with several regions of the patient's face so as to ensure a gas sealing. For instance, the membrane 33 of the cushion 3 can constitute a gas seal along the intermediate nose region (i.e. the area of the nose located at the junction of the bone and cartilage) or the nasal bridge region (i.e. the nasal area above the bone/cartilage junction), the lower lip region area situated under the lower lip or the chin region, and the two opposite lateral regions of the nose and mouth of the patient (i.e. right and left flange regions of the nose and mouth). Of course, other embodiments and shapes are possible.

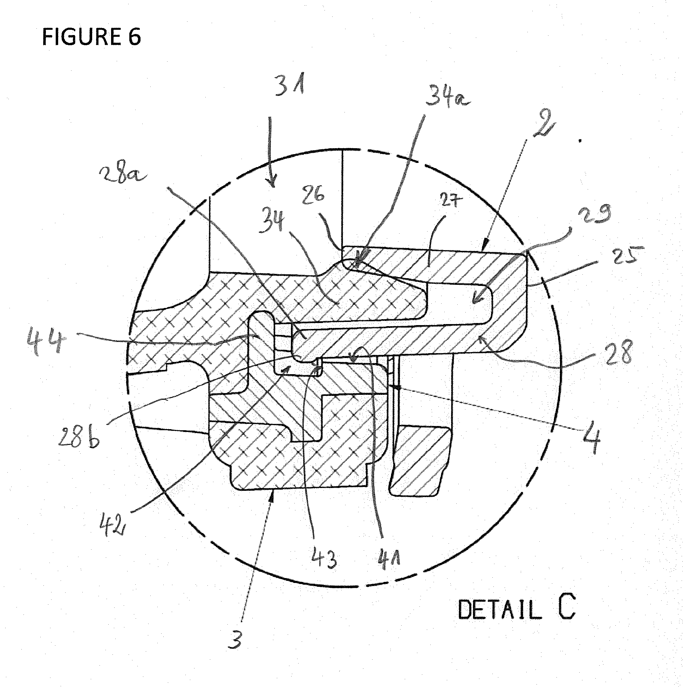

Further, as shown in Figures 5 and 6, the shroud 2 comprises an inner wall 27 and an outer wall 28, spaced apart from each other. The inner wall 27 delimits the central opening 22 of the shroud 2.

The inner wall 27 and the outer wall 28 of the shroud 2 form coaxially-arranged tubular structures comprising an annular chamber 29 arranged between them. The inner wall 27 and the outer wall 28 of the shroud 2 'sandwich' a cylindrical wall forming a tubular collar 34 surrounding the gas orifice 31 of the flexible cushion 3 and projecting outwardly, when said collar 34 is positioned between said inner and outer walls 27, 28 of the shroud 2.

In other words, said annular chamber 29 forms a space between said inner and outer walls 27, 28 of the shroud 2 and the collar 34 of the cushion 3 is inserted into said annular chamber 29 and squeezed between said inner and outer walls 27, 28 of the shroud 2, for ensuring a gas tightness in-between. For obtaining a better sealing, the collar 34 of the cushion 3 comprises an annular bump or boss 34a projecting inwardly, i.e. radially into the central opening 22 of the shroud 2. The annular bump 34a is squeezed between said inner wall 27 and outer wall 28 of the shroud 2, when the shroud 3 is fixed to the ring 4. As the collar 34 of the cushion 3 is made of silicone that is a resilient material, the boss or bump 34a will be deformed so as to conform in shape, i.e. closely match, the surface or profile of the inner and outer walls 27, 28 of the shroud 2 thereby obtaining a good gas sealing in-between.

In other words, the collar 34 is inserted into the annular chamber 29 and is squeezed between the inner wall 27 and outer wall 28 of the shroud 2, thereby ensuring an efficient gas sealing, i.e. gas tightness, between the cushion 3 and the shroud 2 so as to avoid or at least limit gas leaks.

According to the present invention, the shroud 2 is removably attached directly to the ring 4 that is firmly integrated in the body of the flexible cushion 3. This constitutes a positive attachment, such as a male/female connection or the like.

In other words, the ring 4 is positioned between the shroud 2 and the flexible cushion 3 so as to ensure the mechanical tight engagement between the cushion 3 and the shroud 2. Thanks to the presence of the rigid ring 4 between them, the shroud 2 can be quickly and easily fixed directly to the flexible cushion 3 by the patients, especially in the frame of the daily cleaning of the components of the mask.

More precisely, the outer wall 28 of the shroud 2 comprises an annular free end 28a, which comprises one or several abutments 28b, such as teeth or fingers, projecting outwardly. Abutments 28b are designed/configured for being able to grip and hold the rigid ring 4.

Further, the rigid ring 4 comprises an inner annular wall 41 and at least one groove 42, i.e. one or several lodgings, arranged in said an inner annular wall 41, said at least one groove 42 comprises a front border 43, i.e. a groove 42 with a front border 43, forming a seat that can cooperate with an (or several) abutment 28b or tooth of the shroud 2.

In other words, for ensuring a firm attachment of the shroud 2 to the ring 4, at least one abutment 28b of the shroud 2 is lodged into at least one groove 42 of the rigid ring 4 and, at the same time, abuts against the front border 43 of the rigid ring 4, thereby holding the shroud 2 integral with the rigid ring 4.

Preferably, the shroud 2 is, firmly but removably, coupled/attached directly to the rigid ring 4 by means of the teeth or abutments 28b carried by the shroud 2 which cooperates with the grooves 42 front border 43, i.e. lodgings, carried by the rigid ring 4.

As illustrated in Figures 1, 2, 4, 7 and 8, a hollow curved connector 50, i.e. having an elbow shape, is inserted and rotatably retained into the central opening 22 traversing the shroud 2. The hollow curved connector 50 has a general bent tubular-shape, namely a kind of "L"-shape and comprises a lumen or inner passage for conveying gas under pressure, such as air, to the mask 1, in particular to the breathing chamber 30 of the cushion 3.

As detailed in Fig. 7 and 8, the proximal end 53 of the hollow curved connector 50 is inserted into the central opening 22 of the shroud 2. Said proximal end 53 has a cylindrical or tronconical shape and comprises two external lips 54, 55, spaced from each other, projecting outwardly around the outer periphery of the proximal end 53 of the connector 50 so as to form annular abutments. Once inserted into the central opening 22 of the shroud 2, the proximal end 53 of the connector 50 sandwiches the inner wall 27 of the central opening 22 of the shroud 2 between the two external lips 54, 55.

More precisely, the two external lips 54, 55 cooperate with the front 25 and rear 26 surfaces located at the ends of the inner wall 27 of the shroud 2 so as to hold the hollow curved connector 50 in position into the central opening 22 of the shroud 2. In other words, the front 25 and rear 26 surfaces of the shroud 2 abut against the two external lips 54, 55 of the curved connector 50 that form annular abutments so as to hold the connector 50 in place. This constitutes a kind of snap-fit or interlocking connection; however, other types of connection are also useable.

Furthermore, a tubular connecting piece 52, as shown in Figure 1, is used for connecting a gas feeding line, i.e. a flexible hose, to the hollow curved connector 50. The tubular connecting piece 52 can be easily attached to or detached from the hollow curved connector 50. This allows an easy and fast connection and disconnection of the mask 1 from the gas circuit. When the tubular connecting piece 52 is attached to the hollow curved connector 50, it can still rotate with respect to said hollow curved connector 50. The hollow curved connector 50 and the tubular connecting piece 52 connected thereto are generally made of polymer, e.g. plastic.

Generally speaking, the frameless respiratory mask of the present invention, such as a frameless nasal mask, is light and comfortable to wear. Such a simple architecture, i.e. frameless, leads to a reduction of the overall size and weight of the mask, thereby allowing easy assembling/disassembling operations and further efficient positioning and securing of the mask on the patient's face, as well as a good tightness (i.e. seal) and an improved comfort of use for the patient.

Although a frameless nasal mask has been detailed hereabove, the frameless mask of the present invention can also be another type of respiratory mask, such as a facial mask.

Generally speaking, the frameless respiratory mask of the present invention can be used in a method for treatment of a respiratory disorder or condition affecting infant, toddler, child or adult patients.

## Claims

1. Frameless respiratory mask (1) comprising:
- a shroud (2) comprising an upper arm (20) projecting upwardly and two lateral arms (21) projecting laterally, the upper arm (20) and the two lateral arms (21) being arranged around a central opening (22) traversing the shroud (2), the upper arm (20) and the two lateral arms (21) each comprising a free end (23) with headgear connection structures (24) adapted for connecting a headgear thereto,
- a rigid ring (4) traversed by an inner passage (40), and
- a flexible cushion (3) defining a breathing chamber (30), said breathing chamber (30) comprising a gas orifice (31), and a rear opening (32) adapted for receiving at least a part of a patient's face,
and wherein :
- the rigid ring (4) is sandwiched between the shroud (2) and the flexible cushion (3), and
- the gas orifice (31) of the flexible cushion (3) is coaxially arranged and in fluid communication with the central opening (22) of the shroud (2),
**characterized in that**:
i) the rigid ring (4) is integrally and non-removably attached to the flexible cushion (3), and
ii) the shroud (2) is further removably attached directly to the rigid ring (4).

2. Frameless mask according to the preceding Claim, **characterized in that** the shroud (2) is removably coupled directly to the rigid ring (4) and to the flexible cushion (3) by means of connecting structures (41, 28b) cooperating together.

3. Frameless mask according to any one of the preceding Claims, **characterized in that** connecting structures (41, 28b) are carried by the shroud (2) and the rigid ring (4) so as to hold the shroud (2) attached directly to the rigid ring (4).

4. Frameless mask according to any one of the preceding Claims, **characterized in that**:
- the shroud (2) comprises an outer wall (28) comprising an annular free end (28a), said annular free end (28a) comprising at least one abutment (28b) projecting outwardly, and
- the rigid ring (4) comprises an inner annular wall (41) and at least one groove (42) arranged in said an inner annular wall (41), said at least one groove (42) comprising a front border (43),
- said at least one abutment (28b) of the shroud (2) being lodged into said at least one groove (42) of the rigid ring (4) and further abutting against said front border (43) of the rigid ring (4), for holding the shroud (2) integral with the rigid ring (4), when the shroud (2) is attached directly to the rigid ring (4).

5. Frameless mask according to any one of the preceding Claims, **characterized in that** the shroud (2) comprises an inner wall (27) delimiting the central opening (22) and the outer wall (28) comprising an annular free end (28a) carrying the at least one abutment (28b), said inner wall (27) and outer wall (28) being spaced apart from each other, said inner wall (27) and outer wall (28) sandwiching a collar (34) surrounding the gas orifice (31) of the flexible cushion (3).

6. Frameless mask according to Claim 5, **characterized in that** the inner wall (27) and the outer wall (28) of the shroud (2) are coaxially arranged.

7. Frameless mask according to any one of the preceding Claims, **characterized in that** the inner wall (27) and the outer wall (28) of the shroud (2) are spaced by an annular chamber (29), the collar (34) of the cushion (3) being inserted into said annular chamber (29) of the shroud (2) and squeezed between said inner wall (27) and outer wall (28) of the shroud (2), thereby ensuring a gas tightness in-between.

8. Frameless mask according to any one of the preceding Claims, **characterized in that** the collar (34) of the cushion (3) comprises an annular boss (34a) projecting inwardly, said annular boss (34a) being squeezed between said inner wall (27) and outer wall (28) of the shroud (2), when the shroud (3) is fixed to the ring (4).

9. Frameless mask according to any one of the preceding Claims, **characterized in that** the ring (4) is non-removably snap-fitted or similar to the flexible cushion (3).

10. Frameless mask according to any one of the preceding Claims, **characterized in that** the rigid ring (4) and the shroud (2) are made of polymer material.

11. Frameless mask according to any one of the preceding Claims, **characterized in that** the flexible cushion (3) is made of silicone.

12. Frameless mask according to any one of the preceding Claims, **characterized in that** it is :
- a nasal mask comprising a cushion (3) sized for covering at least part of the nose region of a user, when the mask is worn by said user, or
- a facial mask comprising a cushion (3) sized for covering at least part of the nose region and the mouth region of a user, when the mask is worn by said user, or

13. Frameless mask according to any one of the preceding Claims, **characterized in that** it further comprises one or more of the following features :
- the breathing chamber (30) comprises a rear opening (32) adapted for receiving at least a part of a patient's face, said rear opening (32) being bordered at its periphery by a flexible membrane (33),
- a hollow curved connector (50) is fixed to the shroud (2) and rotatable with respect to said shroud (2), preferably the hollow curved connector (x) is detachably fixed to the shroud (2), and/or
- a headgear attached to the headgear connection structures (24) of the holding arm (20) and/or at least one of the two lateral arms (21).

14. Respiratory assembly comprising a gas delivery device and a respiratory frameless mask according to any one of the preceding Claims, preferably the gas delivery device is connected to the respiratory mask by means of a gas line, such as a flexible hose.

## Patentansprüche

1. Rahmenlose Atemmaske (1), umfassend:
- eine Abdeckung (2), die einen oberen Arm (20), der nach oben hervorragt, und zwei seitliche Arme (21), die seitlich hervorragen, umfasst, wobei der obere Arm (20) und die zwei seitlichen Arme (21) um eine zentrale Öffnung (22) angeordnet sind, die durch die Abdeckung (2) verläuft, wobei der obere Arm (20) und die zwei seitlichen Arme (21) jeweils ein freies Ende (23) mit Kopfgestellverbindungsstrukturen (24), die zum Verbinden eines Kopfgestells damit angepasst sind, umfassen,
- einen starren Ring (4), durch den ein interner Durchlass (40) verläuft, und
- ein flexibles Polster (3), das eine Atmungskammer (30) definiert, wobei die Atmungskammer (30) eine Gasöffnung (31) und eine rückwärtige Öffnung (32), die zum Aufnehmen mindestens eines Teils des Gesichts eines Patienten angepasst ist, umfasst,
und wobei:
- der starre Ring (4) zwischen der Abdeckung (2) und dem flexiblen Polster (3) eingeschoben ist und
- die Gasöffnung (31) des flexiblen Polsters (3) mit der zentralen Öffnung (22) der Abdeckung (2) koaxial angeordnet ist und in Fluidverbindung steht,
**dadurch gekennzeichnet, dass**:
i) der starre Ring (4) einstückig und nicht ablösbar an dem flexiblen Polster (3) befestigt ist und
ii) die Abdeckung (2) weiter ablösbar direkt an dem starren Ring (4) befestigt ist.

2. Rahmenlose Maske nach dem vorstehenden Anspruch, **dadurch gekennzeichnet, dass** die Abdeckung (2) mittels miteinander kooperierender Verbindungsstrukturen (41, 28b) ablösbar direkt mit dem starren Ring (4) und mit dem flexiblen Polster (3) gekoppelt ist.

3. Rahmenlose Maske nach einem der vorstehenden Ansprüche, **dadurch gekennzeichnet, dass** Verbindungsstrukturen (41, 28b) von der Abdeckung (2) und dem starren Ring (4) getragen werden, um die Abdeckung (2) direkt an dem starren Ring (4) befestigt zu halten.

4. Rahmenlose Maske nach einem der vorstehenden Ansprüche, **dadurch gekennzeichnet, dass**:
- die Abdeckung (2) eine Außenwand (28), die ein ringförmiges freies Ende (28a) umfasst, umfasst, wobei das ringförmige freie Ende (28a) mindestens einen nach außen hervorragenden Anschlag (28b) umfasst, und
- der starre Ring (4) eine ringförmige Innenwand (41) und mindestens eine in der einen ringförmigen Innenwand (41) angeordnete Nut (42) umfasst, wobei die mindestens eine Nut (42) eine vordere Einfassung (43) umfasst,
- der mindestens eine Anschlag (28b) der Abdeckung (2) in der mindestens einen Nut (42) des starren Rings (4) untergebracht ist und weiter gegen die vordere Einfassung (43) des starren Rings (4) anschlägt, um die Abdeckung (2) mit dem starren Ring (4) einstückig zu halten, wenn die Abdeckung (2) direkt an dem starren Ring (4) befestigt wird.

5. Rahmenlose Maske nach einem der vorstehenden Ansprüche, **dadurch gekennzeichnet, dass** die Abdeckung (2) eine Innenwand (27), die die zentrale Öffnung (22) abgrenzt, und die Außenwand (28), die ein ringförmiges freies Ende (28a) umfasst, das den mindestens einen Anschlag (28b) trägt, umfasst, wobei die Innenwand (27) und die Außenwand (28) voneinander beabstandet sind, wobei zwischen der Innenwand (27) und der Außenwand (28) eine Manschette (34) eingeschoben ist, die die Gasöffnung (31) des flexiblen Polsters (3) umgibt.

6. Rahmenlose Maske nach Anspruch 5, **dadurch gekennzeichnet, dass** die Innenwand (27) und die Außenwand (28) der Abdeckung (2) koaxial angeordnet sind.

7. Rahmenlose Maske nach einem der vorstehenden Ansprüche, **dadurch gekennzeichnet, dass** die Innenwand (27) und die Außenwand (28) der Abdeckung (2) durch eine ringförmige Kammer (29) beabstandet sind, wobei die Manschette (34) des Polsters (3) in die ringförmige Kammer (29) der Abdeckung (2) eingesetzt und zwischen der Innenwand (27) und der Außenwand (28) der Abdeckung (2) zusammengedrückt wird, wodurch dazwischen eine Gasdichtheit gewährleistet wird.

8. Rahmenlose Maske nach einem der vorstehenden Ansprüche, **dadurch gekennzeichnet, dass** die Manschette (34) des Polsters (3) eine ringförmige Auswölbung (34a), die nach innen hervorragt, umfasst, wobei die ringförmige Auswölbung (34a) zwischen der Innenwand (27) und der Außenwand (28) der Abdeckung (2) zusammengedrückt wird, wenn die Abdeckung (3) an dem Ring (4) angebracht wird.

9. Rahmenlose Maske nach einem der vorstehenden Ansprüche, **dadurch gekennzeichnet, dass** der Ring (4) nicht ablösbar in das flexible Polster (3) eingeschnappt wird oder Ähnliches.

10. Rahmenlose Maske nach einem der vorstehenden Ansprüche, **dadurch gekennzeichnet, dass** der starre Ring (4) und die Abdeckung (2) aus Polymermaterial hergestellt sind.

11. Rahmenlose Maske nach einem der vorstehenden Ansprüche, **dadurch gekennzeichnet, dass** das flexible Polster (3) aus Silikon hergestellt ist.

12. Rahmenlose Maske nach einem der vorstehenden Ansprüche, **dadurch gekennzeichnet, dass** sie Folgendes ist:
- eine Nasenmaske, die ein Polster (3) umfasst, das zum Abdecken mindestens eines Teils des Nasenbereichs eines Benutzers dimensioniert ist, wenn die Maske von dem Benutzer getragen wird, oder
- eine Gesichtsmaske, die ein Polster (3) umfasst, das zum Abdecken mindestens eines Teils des Nasenbereichs und des Mundbereichs eines Benutzers dimensioniert ist, wenn die Maske von dem Benutzer getragen wird, oder

13. Rahmenlose Maske nach einem der vorstehenden Ansprüche, **dadurch gekennzeichnet, dass** sie weiter ein oder mehrere der folgenden Merkmale umfasst:
- die Atmungskammer (30) umfasst eine rückwärtige Öffnung (32), die zum Aufnehmen mindestens eines Teils des Gesichts eines Patienten angepasst ist, wobei die rückwärtige Öffnung (32) an ihrem Umfang durch eine flexible Membran (33) begrenzt ist,
- ein hohles gebogenes Verbindungsstück (50) ist an der Abdeckung (2) angebracht und in Bezug auf die Abdeckung (2) drehbar, wobei das hohle gebogene Verbindungsstück (x) bevorzugt abnehmbar an der Abdeckung (2) angebracht ist, und/oder
- ein Kopfgestell an den Kopfgestellverbindungsstrukturen (24) des Haltearms (20) und/oder mindestens eines der zwei seitlichen Arme (21) befestigt.

14. Atmungsmontage, umfassend eine Gaszuführvorrichtung und eine rahmenlose Atemmaske nach einem der vorstehenden Ansprüche, wobei die Gaszuführvorrichtung bevorzugt mittels einer Gasleitung, wie beispielsweise eines flexiblen Schlauchs, mit der Atemmaske verbunden ist.

## Revendications

1. Masque respiratoire sans cadre (1) comprenant :
- un dispositif de protection (2) comprenant un bras supérieur (20) faisant saillie vers le haut et deux bras latéraux (21) faisant saillie latéralement, le bras supérieur (20) et les deux bras latéraux (21) étant agencés autour d'une ouverture centrale (22) traversant le dispositif de protection (2), le bras supérieur (20) et les deux bras latéraux (21) comprenant chacun une extrémité libre (23) comportant des structures de connexion à un harnais (24) adaptées pour connecter un harnais à celui-ci,
- un anneau rigide (4) traversé par un passage interne (40), et
- un coussin flexible (3) définissant une chambre respiratoire (30), ladite chambre respiratoire (30) comprenant un orifice de gaz (31), et une ouverture arrière (32) adaptée pour recevoir au moins une partie d'un visage d'un patient,
Et dans lequel :
- l'anneau rigide (4) est pris en sandwich entre le dispositif de protection (2) et le coussin flexible (3), et
- l'orifice de gaz (31) du coussin flexible (3) est agencé de manière coaxiale et en communication fluide avec l'ouverture centrale (22) du dispositif de protection (2),
**caractérisé en ce que** :
i) l'anneau rigide (4) est attaché d'un seul tenant et de manière non amovible au coussin flexible (3), et
ii) le dispositif de protection (2) est en outre attaché de manière amovible directement à l'anneau rigide (4).

2. Masque sans cadre selon la revendication précédente, **caractérisé en ce que** le dispositif de protection (2) est couplé de manière amovible directement à l'anneau rigide (4) et au coussin flexible (3) au moyen de structures de connexion (41, 28b) coopérant ensemble.

3. Masque sans cadre selon l'une quelconque des revendications précédentes, **caractérisé en ce que** les structures de connexion (41, 28b) sont portées parle dispositif de protection (2) et l'anneau rigide (4) de façon à maintenir le dispositif de protection (2) attaché directement à l'anneau rigide (4).

4. Masque sans cadre selon l'une quelconque des revendications précédentes, **caractérisé en ce que** :
- le dispositif de protection (2) comprend une paroi externe (28) comprenant une extrémité annulaire libre (28a), ladite extrémité annulaire libre (28a) comprenant au moins une butée (28b) faisant saillie vers l'extérieur, et
- l'anneau rigide (4) comprend une paroi annulaire interne (41) et au moins une gorge (42) agencée dans ladite une paroi annulaire interne (41), ladite au moins une gorge (42) comprenant une bordure avant (43),
- ladite au moins une butée (28b) du dispositif de protection (2) étant logée dans ladite au moins une gorge (42) de l'anneau rigide (4) et étant en outre en butée contre ladite bordure avant (43) de l'anneau rigide (4), pour maintenir le dispositif de protection (2) d'un seul tenant avec l'anneau rigide (4), lorsque le dispositif de protection (2) est attaché directement à l'anneau rigide (4).

5. Masque sans cadre selon l'une quelconque des revendications précédentes, **caractérisé en ce que** le dispositif de protection (2) comprend une paroi interne (27) délimitant l'ouverture centrale (22) et la paroi externe (28) comprenant une extrémité annulaire libre (28a) portant l'au moins une butée (28b), lesdites paroi interne (27) et paroi externe (28) étant espacées l'une de l'autre, lesdites paroi interne (27) et paroi externe (28) prenant en sandwich un collier (34) entourant l'orifice de gaz (31) du coussin flexible (3).

6. Masque sans cadre selon la revendication 5, **caractérisé en ce que** la paroi interne (27) et la paroi externe (28) du dispositif de protection (2) sont agencées de manière coaxiale.

7. Masque sans cadre selon l'une quelconque des revendications précédentes, **caractérisé en ce que** la paroi interne (27) et la paroi externe (28) du dispositif de protection (2) sont espacées par une chambre annulaire (29), le collier (34) du coussin (3) étant inséré dans ladite chambre annulaire (29) du dispositif de protection (2) et comprimé entre lesdites paroi interne (27) et paroi externe (28) du dispositif de protection (2), assurant ainsi une étanchéité aux gaz entre celles-ci.

8. Masque sans cadre selon l'une quelconque des revendications précédentes, **caractérisé en ce que** le collier (34) du coussin (3) comprend une protubérance annulaire (34a) faisant saillie vers l'intérieur, ladite protubérance annulaire (34a) étant comprimée entre lesdites paroi interne (27) et paroi externe (28) du dispositif de protection (2), lorsque le dispositif de protection (3) est fixé à l'anneau (4).

9. Masque sans cadre selon l'une quelconque des revendications précédentes, **caractérisé en ce que** l'anneau (4) est encliqueté de manière non amovible ou similaire sur le coussin flexible (3).

10. Masque sans cadre selon l'une quelconque des revendications précédentes, **caractérisé en ce que** l'anneau rigide (4) et le dispositif de protection (2) sont faits d'un matériau polymère.

11. Masque sans cadre selon l'une quelconque des revendications précédentes, **caractérisé en ce que** le coussin flexible (3) est fait de silicone.

12. Masque sans cadre selon l'une quelconque des revendications précédentes, **caractérisé en ce qu'**il s'agit :
- d'un masque nasal comprenant un coussin (3) dimensionné pour recouvrir au moins une partie de la région du nez d'un utilisateur, lorsque le masque est porté par ledit utilisateur, ou
- d'un masque facial comprenant un coussin (3) dimensionné pour recouvrir au moins une partie de la région du nez et de la région de la bouche d'un utilisateur, lorsque le masque est porté par ledit utilisateur.

13. Masque sans cadre selon l'une quelconque des revendications précédentes, **caractérisé en ce qu'**il comprend en outre un ou plusieurs des éléments suivants :
- la chambre respiratoire (30) comprend une ouverture arrière (32) adaptée pour recevoir au moins une partie d'un visage d'un patient, ladite ouverture arrière (32) étant bordée au niveau de sa périphérie par une membrane flexible (33),
- un connecteur incurvé creux (50) est fixé au dispositif de protection (2) et peut tourner par rapport audit dispositif de protection (2), de préférence le connecteur incurvé creux (x) est fixé de manière détachable au dispositif de protection (2), et/ou
- un harnais attaché aux structures de connexion à un harnais (24) du bras de maintien (20) et/ou d'au moins l'un des deux bras latéraux (21).

14. Ensemble respiratoire comprenant un dispositif ds'administration de gaz et un masque respiratoire sans cadre selon l'une quelconque des revendications précédentes, de préférence le dispositif d'administration de gaz est connecté au masque respiratoire au moyen d'une conduite de gaz, telle qu'un tuyau flexible.
